# EUROPEAN PATENT SPECIFICATION

(11) **EP 0 883 615 B1**
(45) Date of publication and mention of the grant of the patent: **23.10.2002**
(21) Application number: 96940676.8
(22) Date of filing: 12.12.1996
(51) Int. Cl.: C07D 307/60

(54) **ADDITIVES USABLE IN PREPARATION OF ALKENYL SUCCINIC ANHYDRIDE**
ADDITIVE ZUR HERSTELLUNG VON ALKENYL-BERNSTEINSÄUREANHYDRID
ADDITIFS UTILES POUR LA PREPARATION D'ANHYDRIDES D'ACIDES ALCENYL-SUCCINIQUES

(30) Priority: 22.12.1995 FI 956220
(43) Date of publication of application: 16.12.1998
(73) Proprietor: KEMIRA CHEMICALS OY, 00100 Helsinki (FI)
(72) Inventor: MÄKIPEURA, Petri, FIN-06850 Kulloo (FI); KAPANEN, Mika, FIN-06100 Porvoo (FI); TULISALO, Jukka, FIN-04230 Kerava (FI); KOSKIMIES, Salme, FIN-00850 Helsinki (FI)
(74) Representative: Hansen, Bernd, Dr. Dipl.-Chem.
(86) International application number: FI9600657
(87) International publication number: WO97023474

(56) References cited:
- EP-A- 0 359 316
- US-A- 3 546 184
- US-A- 3 952 023
- US-A- 4 156 686
- PATENT ABSTRACTS OF JAPAN, Vol. 6, No. 102, C-107; & JP,A,57 032 276 (DAINIPPON INK KAGAKU KOGYO K.K.), 20 February 1982.
- JOURNAL OF POLYMER SCIENCE, Volume 26, 1988, NORMAN G. GAYLORD et al., "Radical-Catalyzed Homopolymerization of Maleic Anhydride in Presence of Polar Organic Compounds", pages 1903-1909.

## Description

The present invention relates to a process for the preparation of an alkenyl succinic anhydride from an olefinically unsaturated hydrocarbon selected from a straight chain or branched internal mono-olefin having 16-20 carbon atoms and any mixtures thereof and a maleic anhydride by using additives which improve the yield and reduce side reactions.

Alkenyl succinic anhydrides, or ASAs, are reaction products of olefins and maleic anhydride (MHA). They are produced by the ene reaction, which is an indirect substituting addition from a compound with an electron-poor double bond (enophil) to a compound which contains an allylic hydrogen (ene). In the preparation of ASA, the enophil component is MHA and the ene component is olefin. In the reaction, the double bond of the olefin shifts to the subsequent carbon, a new bond is formed between the olefin and MHA, and the allylic hydrogen present in the olefin shifts to MHA.

The ene reaction requires a relatively high temperature, since its activation energy is high, i.e. approx. 20 kcal/mol. The reaction speed of the reaction also increases strongly as a function of the temperature, and thus it is preferable to use a relatively high reaction temperature in order for the product to form at a reaction speed which is at least satisfactory. For the above reasons, ASA is produced at relatively high temperatures, and thereby also the probability of the occurrence of various side reactions increases. Side reactions observed in the preparation of ASA include the polymerization of MHA, the copolymerization of olefin and MHA, and the oligomerization of olefin. It has also been observed in the preparation of ASA that further reactions of ASA occur, which include further reaction of ASA with MHA and the retroene reaction, in which ASA breaks down to its initial substances. Thermal decomposition of MHA and ASA can also be observed in the preparation of ASA. The side and decomposition reactions lead to a poor yield and a poor quality of the actual product, i.e. the forming product often has a strong color and contains large amounts of various polymeric and tarry compounds.

Attempts are made, by using various additives, to reduce the side reactions occurring in the preparation. The purpose of the additives is to lead to a purer and more monomeric product having less color. This is done for the reason that ASAs often have a high molecular weight, and in practice they are impossible to purify, for example, by distillation. The additives may work through various mechanisms, for example through the catalysis of the principal reaction or the inhibition of the side reactions but, with respect to many of the additives used in the preparation of ASA, the way in which the additive works is not known with full certainty.

Alkenyl succinic anhydrides may be prepared from olefins of highly different lengths, ranging from propylene to polymers having hundreds of carbon atoms. ASAs prepared from polymers, mainly polyisobutene, are used as dispersants and corrosion inhibitors in additives of diesel and gasoline motors. The molecular weight of the polymer used usually ranges from 900 to 1500 g/mol, and the polymer is prepared from isobutene.

ASA may also be prepared from shorter-chain olefins, as pointed out above. Of shorter-chain olefins, mainly 16-20 carbon atom a-olefins are used, which may have been isomerized before the preparation of ASA. In addition, C₆-C₁₄ a-olefins and C₁₅-C₁₈ internal olefins are used on a larger scale. ASAs prepared from these olefins are used mostly as reactive paper-sizing chemicals. The ASAs used for paper sizing are prepared from C₁₆-C₂₀ α-olefins, which are isomerized, and thus an ASA which is liquid at room temperature is obtained. In paper making, the anhydride group present in ASA reacts with the hydroxyl group of cellulose, and the alkenyl chain forms on the surface of paper or cardboard a hydrophobic surface which prevents water and ink from penetrating into the paper. The amount of ASA used in paper sizing will increase, since in paper making a shift is being made to paper making in alkaline conditions, in which conventional hydrophobic sizes cannot be used.

Starch derivatives of ASA, prepared from even shorter-chain olefins, such as octenes, are used as coagulants in foods, juices and puddings. Sodium and potassium salts of octenyl succinic anhydride for their part are used as surfactants in industrial metal-cleaning and floor-cleaning agents. ASAS made from α-C₁₂-C₁₈ are used as leather-finishing substances to soften leather and to protect it from water. ASAs made from these olefins may also be used as auxiliaries in epoxy resin setting agents, whereby better electrical and flexibility properties are obtained for the resin, and as additives of oil and as corrosion inhibitors. ASA can also be used for preparing various ester, amide, imide, and other derivatives, which are used partly in applications similar to those in which actual ASA is used, for example as additives in oil and as corrosion inhibitors.

ASAs are prepared by the ene reaction from olefins and maleic anhydride. The reaction requires a high temperature, 150-300 °C. Side reactions occurring at this temperature include polymerization of MHA, thermal decomposition of MHA, copolymerization of MHA and olefin, and oligomerization of olefin. The side reactions cause the efficiency of the reaction to deteriorate, which in turn leads to a poor yield of ASA and an impure product. Below, there are presented various additives used in the preparation of ASA in order to inhibit side reactions.

According to patent publications US 3 412 111 and EP 359 316, attempts have been made to reduce polymerization in the preparation reactions by using hydroxy or aminoaromatic compounds as additives in the preparation of ASA. The initial olefin used in the examples of the said patent publications is dodecene or a mixture of dodecene and tetradecene. In patent publication US 3 476 774, sterically hindered phenols, such as 2,6-di-t-butyl-4-methylphenol (BHT), are used for reducing the breaking down of MHA. In the examples of the patent publication, ASAs were prepared from polyisobutene, and in some of the experiments an organic solvent, such as xylene, was also used. However, a good ASA yield is not obtained by using the additives used in these patent publications, and the product obtained contains varying amounts of polymeric compounds. Furthermore, a product of unsuitable color is obtained with some of the additives mentioned in the patent publications.

In patent publication US 4 599 433, the additives used in the preparation of ASA are alkoxides of Ti, Zr, V or Al, of which C₂-C₄ alkoxides would be recommendable. It has been observed that the additives mentioned in the patent publication increase the yield of ASA and reduce the polymerization of MHA. In the examples of the patent publication, ASA is prepared by using polyisobutene and 2,4,4-trimethylpentene, and as the additive titanium butoxide. ASAs prepared in accordance with the patent publication can, according to the publication, be used as anticorrosive agents and as additives in oil.

In patent publication US 4 761 488, aluminum acetylacetonate is used as an additive in the preparation of ASA to reduce the formation of a black solid, and it has been found to improve considerably the color of the ASA formed. The olefin used in the examples of the patent publication is tetradecene or C₁₅-C₂₀ internal olefins. ASAs prepared in accordance with the patent publication are used, according to the publication, as anticorrosive agents.

However, as later examples show (Comparative Examples 3, 4, 14 and 21), a high yield of ASA and a polymer-free product have not been achieved by using the additives according to the said patent publications US 4 599 433 and US 4 761 488.

When ASA is prepared in accordance with patent publication Us 4 431 825 from olefins (mainly polyisobutenes having Mₙ = 300-3000), the preparation reaction can be catalyzed with iron or tin chlorides, iron bromide or phosphoric acid. However, the catalyzing effect of iron chloride was not observed in Examples 5 and 19. It is disclosed in patent publication US 3 935 249 that by using as additives inorganic compounds, preferably compounds containing chlorine or bromine, it is possible to reduce the formation of tarry byproducts which are formed because of polymerization, and to reduce the thermal decomposition of MHA. The problem involved with the additives according to these patent publications consists of various halogenic compounds, which may be environmentally hazardous, and, for example, chlorine is a highly corrosive chemical.

According to patent publication EP 317 004, various alkyl zincs and zinc alkoxides can be used as additives in the preparation of ASA in order to reduce side reactions appearing in the preparation. In the experiments in the examples of the patent publication, these additives did not improve the yield of ASA as compared with ASA prepared without the additive, only the color of the product improved somewhat. The olefins used in the examples of the patent publication were C₁₄-C₂₀ olefins, which may be either α-olefins or internal linear or branched olefins. According to the publication, ASAs prepared in accordance with the patent publication are used as anticorrosive agents.

In patent publications US 4 958 034 and US 5 021 169, aryl fluorophosphates and tri-orthoalkylphenyl phosphates are used as additives. In these patent publications, attention has been paid to the color of the product formed and to tar formation, but not to the yield with which ASA can be prepared. In the examples of both patent publications, ASA is prepared from C₁₆-C₁₈ α-olefins which have been rendered internal by isomerization. However, an ASA prepared with the additives according to the patent publication has contained polymeric compounds almost in an amount of 10 %, depending on the additive used.

In patent publication US 4 581 464, a C₈-C₁₈ alkyl succinic anhydride is used as an additive in the preparation of ASA. By means of this additive, the initial substances used in the reaction, i.e. MHA and a-olefin, have together been rendered a homogenous mixture, which reduces the formation of undesirable side reactions. The reaction mixture must contain alkyl succinic anhydride in an amount of at least 20 %. The use of this additive is uneconomical, since it is impossible to separate from the end product.

In patent publication US 4 388 471, furan-type compounds are used as an additive in the preparation of ASA. These compounds are believed to react with MHA through a reversible cycloaddition, the addition product then breaking down under heat. Thus the amount of free MHA in the reaction mixture is as small as possible, and this reduces the polymerization of MHA. But the amount of the necessary furan-type compound in the reaction mixture must be large in order to lead to the desired end result. The compound used must be removed before the end use of ASA, and thus it is uneconomical to use such additives.

In all of the above-mentioned patents and processes there are various problems in the preparation of ASA. They often lead to a poor yield of the product, and the end products still contain large amounts of polymers, or the processes are in other respects unsuitable and uneconomical. We have now found additives by means of which ASA prepared from a straight chain or branched internal mono-olefin having a carbon chain of 16-20 carbon atoms, or any mixture thereof, and a maleic anhydride can be prepared considerably more efficiently than by using prior additives, in such a manner that the yield of ASA can be maximized and the formation of various polymers can be minimized. These additives provide a high ASA yield, and the losses of initial materials when they are used are considerably lower than when prior known additives are used.

The alkenyl succinic anhydride, i.e. ASA, is prepared by the ene reaction at a temperature of 150-250 °c from olefin and maleic anhydride. Any of the new additives cited below can be used in the preparation in order to increase the yield of ASA, to reduce initial-material losses and side reactions, to minimize the amount of polymers formed, and to inhibit the thermal decomposition of MHA and ASA.

It has been found that various benzoquinonic compounds, such as p-naphthobenzoquinone and 2,5-hydroxy-p-benzoguinone, are additives which function especially well in the preparation reaction. Such compounds are added in an amount of 0.05-2.0 mol % of the amount of MHA used.

When acetyl acetonates of transition metals or their oxides, such as molybdenyl acetylacetonate, are used as additives in the preparation of ASA, it is observed that the yield of ASA increases and the losses of initial materials decrease. Acetylacetonates of these metals/metal oxides are added to the reaction mixture in an amount of 0.01-1.0 mol % of the amount of MHA used in the reaction.

It was observed that, in addition to the above, also dialkylsulfoxides, in particular dimethylsulfoxide, considerably improve the yield of ASA and reduce side reactions. These compounds are added in the preparation of ASA to the reaction mixture between olefin and MHA in an amount of 0.05-1.5 mol % of the amount of olefin used.

The olefin used in the preparation may be by structure an internal straight-chain olefin, an internal branched olefin, or any mixture of these.

The straight-chain and branched internal olefins used in the preparation are prepared either by oligomerization or dimerization of olefin monomers, by dehydrogenation of alcohols to olefins, by isomerization of α-olefins, by a metathesis reaction, or the olefin may have been prepared by pyrolysis, at a temperature of 300-600 degrees, from a recycled high-molecular-weight polyethylene waste or other plastic.

The oligomerization may be carried our by using a batch, semi-batch or continuous-working process. The oligomerization catalyst used may be a homogenous Lewis acid catalyst, such as a BF₃ alcohol or BF₃ acid complex or an Al₃Cl₃-HCI, Al₃Cl₂CH₂CH₃ or Al₃Cl₃ aromatic catalyst. The heterogenous oligomerization catalyst used, for its part, may be a synthetic aluminum silica or zeolite.

ASA is prepared at a temperature of 150-250 °C in either a batch reactor or a semi-batch reactor. When ASA is prepared in a semi-batch reactor, MHA is added, to the olefin and any additive according to the invention, in several batches or as a continuous feed at either an even or variable rate, as disclosed, for example, in the claims of patent publication US 4 414 397. It is recommendable to use a reaction temperature of 180-230 °C to improve the efficiency of the reaction. The reaction period may be 0.5-24 hours. The ratio of olefin to MHA may vary within 0.1-5.0/1 (mol/mol), depending on the reaction temperature, reaction period, reaction type, and additive used. The amount of additive in the reaction mixture is for its part also dependent on the reaction temperature, reaction period, reaction type, and the additive used, so that the additive according to the invention is added to the reaction mixture in concentrations in accordance with the invention. Finally any unreacted initial substances are removed from the reaction mixture by vacuum distillation, and an end product (ASA) which is so pure that it need not be purified before use is obtained with a good yield.

In the following examples, additives according to the invention for use in the preparation of ASA are described and are compared with prior known additives.

### Example 15.

101.0 g of a C₁₅-C₁₈ internal straight-chain olefin, 33.7 g of MHA and 0.3 g of 1,2,5,8-tetrahydroxyanthraquinone were weighed into a 300 ml Parr pressure reactor. The reactor was heated to 230 °C, at which temperature it was maintained for 2 hours. The reactor was cooled and opened. The product mixture was analyzed by liquid chromatography, and the ASA yield obtained was 66.9 mol % of the theoretical maximum.

### Example 18.

15 g of a C₁₄ α-olefin, 15 g of a C₁₆ α-olefin, 15 g of a C₁₈ α-olefin, 55 g of an internal straight-chain C₁₅-C₁₈ olefin, and 36.2 g of MHA were weighed into a 300 ml Parr pressure reactor. 0.3 g of 2,5-dihydroxy-para-benzoquinone was added as an additive to the reaction mixture. The reactor was heated to 210 °C, at which temperature it was maintained for 4 hours. After the reaction the reaction mixture contained ASA 61.9 wt.% and unreacted olefin 32.7 wt.%.

### Example 19. (reference)

Example 19 was performed in conditions and with olefins corresponding to those in Example 18, but so that the additive used was FeCl₃, which was an additive according to the claim of US 4,431,825. After the reaction the reaction mixture contained ASA 58.5 wt.% and olefin 24.7 wt.%. That the amount of olefin was small was evidently due to the additive having oligomerized some of the olefin.

### Example 20.

100.9 g of an olefin which had been prepared by oligomerizing a mixture of 1- and 2-butene (so-called raffinate II stream) by using a synthetic aluminum silica catalyst was weighed into a 300 ml Parr pressure reactor. The fraction used contained a C₁₄-C₁₆ fraction separated by distillation. 42.2 g of maleic anhydride was added into the reactor, and 0.2 g of molybdenyl acetylacetonate was weighed as an additive to the mixture. The reactor was heated to 210 °C, at which temperature it was maintained for 4 hours. After the reaction the reaction mixture contained ASA 59.8 wt.% and olefin 28.2 wt.%.

### Example 21. (comparative)

Example 21 was performed in a manner corresponding to Example 20, except that the additive used was titanium butoxide, which is an additive according to patent publication US 4,599,433. After the reaction the reaction mixture contained ASA 58.2 wt.% and olefin 29.2 wt.%.

### Example 22.

104.3 g of an olefin which had been obtained from the pyrolysis of polyethylene and from which the fraction containing C₁₂-C₁₈ had been separated by distillation, 38.1 g of MHA and, as an additive, 0.3 g of p-naphthohydroquinone were weighed into a 300 g Parr pressure reactor. The reactor was heated to 210 °C, at which temperature it was maintained for 4 hours. After the reaction the reaction mixture contained ASA 35.6 wt.% and olefin 39.3 wt.%. That the amount of olefin was large and also that the amount of ASA was small were due to the fact that the olefin obtained from pyrolysis also contained alkanes.

### Example 23. (comparative)

Example 23 was performed in a manner corresponding to Example 22, except that the additive used was 2,6-ditertbutyl-4--methylphenol, which is an additive according to patent publication US 3,412,111. After the reaction the reaction mixture contained ASA 33.2 wt.% and olefin 40.5 wt.%.

## Claims

1. Process for the preparation of alkenyl succinic anhydride from
i) a mono-olefinically unsaturated hydrocarbon selected from a straight chain or branched internal olefin, or any mixture thereof, each having a carbon chain of 16 to 20 carbon atoms, and
ii) a maleic anhydride
characterized that in the reaction a benzoquinone derivative, a dialkylsulfoxide, or an acetylacetonate of a transition metal or a transition metal oxide is used as an additive.

2. A process according to Claim 1, **characterized in that** the additive is 1,4-naphthobenzoquinone.

3. A process according to Claim 1, **characterized in that** the additive is 2,5-dihydroxy-1,4-naphthobenzoguinone.

4. A process according to Claim 1, **characterized in that** the additive is molybdenyl acetylacetonate.

5. A process according to Claim 1, **characterized in that** the additive is dimethylsulfoxide.

6. A process according to Claim 1, **characterized in that** the additive is 1,2,5,8-tetrahydroxyanthraquinone.

7. A process according to Claim 1, **characterized in that** in the reaction mixture the molar ratio of the initial substances is 0.1-5.0 mol of olefinic hydrocarbon to 1.0 mol of maleic anhydride.

8. A process according to Claim 1, **characterized in that** the reaction temperature is 150-250 °C.

9. A process according to Claim 1, **characterized in that** the additive is used in an amount of 0.01-2.0 mol % of the amount of maleic anhydride.

10. A process according to Claim 1, **characterized in that** the olefinically unsaturated hydrocarbon is a straight-chain a-olefin or a straight-chain or branched internal olefin, or a mixture of these.

11. A process according to Claim 1, **characterized in that** the reaction is performed in a batch reactor or a semi-batch reactor.

12. A process according to Claim 11, **characterized in that** in a semi-batch reaction the maleic anhydride and the additive are added in several batches or as a continuous feed in which the feeding rate is even or variable.

13. The use of an alkenyl succinic anhydride prepared by the process according to any of the above claims as an additive in foods, as hydroxy sizing of paper, or as a dispersing agent in lubricants.

## Patentansprüche

1. Verfahren zur Herstellung von Alkenylsuccinsäureanhydrid aus
(i) einem monoolefinisch ungesättigten Kohlenwasserstoff, ausgewählt aus einem unverzweigten oder verzweigten internen Olefin oder einer beliebigen Mischung daraus, die jeweils eine Kohlenstoffkette von 16 bis 20 Kohlenstoffatomen aufweisen, und
(ii) einem Maleinsäureanhydrid,
**dadurch gekennzeichnet, dass** in der Reaktion ein Benzochinonderivat, ein Dialkylsulfoxid oder ein Acetylacetonat eines Übergangsmetalls oder eines Übergangsmetalloxids als Zusatzstoff verwendet wird.

2. Verfahren gemäss Anspruch 1, **dadurch gekennzeichnet, dass** der Zusatzstoff 1,4-Naphthobenzochinon ist.

3. Verfahren gemäss Anspruch 1, **dadurch gekennzeichnet, dass** der Zusatzstoff 2,5-Dihydroxy-1,4-naphthobenzochinon ist.

4. Verfahren gemäss Anspruch 1, **dadurch gekennzeichnet, dass** der Zusatzstoff Molybdänylacetylacetonat ist.

5. Verfahren gemäss Anspruch 1, **dadurch gekennzeichnet, dass** der Zusatzstoff Dimethylsulfoxid ist.

6. Verfahren gemäss Anspruch 1, **dadurch gekennzeichnet, dass** der Zusatzstoff 1,2,5,8-Tetrahydroxyanthrachinon ist.

7. Verfahren gemäss Anspruch 1, **dadurch gekennzeichnet, dass** in der Reaktionsmischung das Molverhältnis der anfänglichen Substanzen 0,1-5,0 mol olefinischer Kohlenwasserstoff zu 1,0 mol Maleinsäureanhydrid beträgt.

8. Verfahren gemäss Anspruch 1, **dadurch gekennzeichnet, dass** die Reaktionstemperatur 150-250°C beträgt.

9. Verfahren gemäss Anspruch 1, **dadurch gekennzeichnet, dass** der Zusatzstoff in einer Menge von 0,01-2,0 mol-%, bezogen auf die Menge an Maleinsäureanhydrid, verwendet wird.

10. Verfahren gemäss Anspruch 1, **dadurch gekennzeichnet, dass** der olefinisch ungesättigte Kohlenwasserstoff ein unverzweigtes α-Olefin oder ein unverzweigtes oder verzweigtes internes Olefin oder eine Mischung daraus ist.

11. Verfahren gemäss Anspruch 1, **dadurch gekennzeichnet, dass** die Reaktion in einem diskontinuierlichen oder semi-diskontinuierlichen Reaktor durchgeführt wird.

12. Verfahren gemäss Anspruch 11, **dadurch gekennzeichnet, dass** in einer semi-diskontinuielichen Reaktion das Maleinsäureanhydrid und der Zusatzstoff in mehreren Chargen oder als kontinuierliche Einspeisung, wobei die Zuführgeschwindigkeit gleichmässig oder variabel ist, zugegeben werden.

13. Verwendung eines nach dem Verfahren gemäss mindestens einem der obigen Ansprüche hergestellten Alkenylsuccinsäureanhydrids als Zusatzstoff in Lebensmitteln, als Hydroxy-Schlichtmittel für Papier oder als Dispersionsmittel in Gleitmitteln.

## Revendications

1. Procédé de préparation d'anhydride alcényle succinique à partir de
(i) un hydrocarbure insaturé mono oléfine choisi parmi une oléfine interne à chaîne linéaire ou ramifiée ou tout mélange de celles-ci, chacune ayant une chaîne carbonée comportant 16 à 20 atomes de carbone, et
(ii) un anhydride maléique
**caractérisé en ce que** dans la réaction, un dérivé de la benzoquinone, un dialkyle sulfoxyde ou un acétyl-acétonate d'un métal de transition ou d'un oxyde de métal de transition est utilisé comme additif.

2. Procédé selon la revendication 1, **caractérisé en ce que** l'additif est la 1,4-naphtobenzoquinone.

3. Procédé selon la revendication 1, **caractérisé en ce que** l'additif est la 2,5-dihydroxy-1,4-naphtobenzoquinone.

4. Procédé selon la revendication 1, **caractérisé en ce que** l'additif est l'acétylacétonate de molybdényle.

5. Procédé selon la revendication 1, **caractérisé en ce que** l'additif est le diméthylsulfoxyde.

6. Procédé selon la revendication 1, **caractérisé en ce que** l'additif est la 1,2,5,8-tétrahydroxyanthraquinone.

7. Procédé selon la revendication 1, **caractérisé en ce que** dans le mélange réactionnel le rapport molaire des substances initiales est de 0,1-5,0 mol d'hydrocarbure oléfinique pour 1,0 mol d'anhydride maléique.

8. Procédé selon la revendication 1, **caractérisé en ce que** la température de la réaction est de 150 à 250 °C.

9. Procédé selon la revendication 1, **caractérisé en ce que** l'additif est utilisé en une quantité de 0,01 à 2,0 % mol de la quantité d'anhydride maléique.

10. Procédé selon la revendication 1, **caractérisé en ce que** l'hydrocarbure oléfinique insaturé est une a-oléfine à chaîne linéaire ou une oléfine interne à chaîne linéaire ou ramifiée ou encore un mélange de celles-ci.

11. Procédé selon la revendication 1, **caractérisé en ce que** la réaction est réalisée dans un réacteur discontinu ou dans un réacteur semi-continu.

12. Procédé selon la revendication 11, **caractérisé en ce que** dans le réacteur semi-continu l'anhydride maléique et l'additif sont ajoutés en plusieurs lots ou en alimentation continue, pour laquelle le taux d'alimentation est constant ou variable.

13. Utilisation d'un anhydride alcényle succinique préparé par le procédé selon l'une quelconque des revendications précédentes comme additif alimentaire, dans le collage hydroxy de papier, ou comme agent dispersant dans les lubrifiants.
